(19) Europäisches Patentamt
European Patent Office
Office européen des brevets

(11) Publication number: **0 556 838 A1**

## EUROPEAN PATENT APPLICATION

(21) Application number: **93102590.2**

(22) Date of filing: **19.02.93**

(51) Int. Cl.5: **C12P 19/12**, C12N 1/16

(30) Priority: **21.02.92 JP 35385/92**

(43) Date of publication of application:
**25.08.93 Bulletin 93/34**

(84) Designated Contracting States:
**AT BE CH DE DK ES FR GB GR IE IT LI LU NL PT SE**

(71) Applicant: **TAKEDA CHEMICAL INDUSTRIES, LTD.**
**1-1, Doshomachi 4-chome**
**Chuo-ku, Osaka 541(JP)**

(72) Inventor: **Miyazaki, Junichi**
**505, 12-1 Matsushiro 3-chome**
**Tsukuba, Ibaraki 305(JP)**
Inventor: **Miyagawa, Kenichiro**
**6-11, Higashitokiwadai 6-chome, Toyono-cho**
**Toyono-gun, Osaka 663-01(JP)**
Inventor: **Sugiyama, Yoshio**
**18-3, Iho 3-chome, Takasago**
**Hyogo 676(JP)**

(74) Representative: **von Kreisler, Alek,**
**Dipl.-Chem. et al**
**Patentanwälte Von Kreisler-Selting-Werner,**
**Postfach 10 22 41, Bahnhofsvorplatz 1**
**D-50462 Köln (DE)**

(54) **Method of producing trehalose.**

(57) The object of the present invention is to provide a method of producing trehalose, wherein a yeast of the genus *Filobasidium* capable of producing trehalose is cultured in a medium to produce and accumulate trehalose in the culture, which trehalose is then harvested from said culture

The use of the present method offers markedly increased accumulation of trehalose and facilitates isolation and purification of trehalose.

EP 0 556 838 A1

[SUMMARY OF THE INVENTION]

The present invention relates to a new method of producing trehalose (O-α-D-glucopyranosyl-(1→1)-α-Dglucopyranoside) by fermentation using microorganisms. Trehalose occurs widely in microorganisms, algae, plants and animals (insects), and is known to serve as an energy storage substance for these organisms. Beyond this, trehalose has recently been found to exhibit protective action against damages of cells and intracellular high molecular substances caused by freezing or drying. This finding triggered preservative application of trehalose in a variety of fields including foods, pharmaceuticals and cosmetics. It is therefore very significant, from the industrial viewpoint, to produce trehalose in large amounts at low cost.

[BACKGROUND]

Known methods of trehalose production include the method based on extraction from baker's yeast [Journal of American Chemical Society, 72, 2059 (1950)], the method using an ascomycetous yeast of the genus *Hansenula* [East German Patent No. 266584], the method based on extraction from fungi of the genus *Rhizoctonia* or *Sclerotium* [Japanese Patent Open to Public Inspection No. 130084/1991], and the fermentation method using a bacterium of the genus *Arthrobacter* [Agricultural and Biological Chemistry, 33, 190 (1969)], but none of these methods are industrially advantageous because the amount of trehalose accumulated is low both intracellularly and extracellularly. Also known is the method using two enzymes (maltose phosphorylase and trehalose phosphorylase) to convert the starting material maltose to trehalose [Japanese Patent Examined Publication No. 60998/1988], but this method has drawbacks such as the high cost of enzyme preparation. There is no established method of producing trehalose at low cost and in large amounts.

[DETAILED DESCRIPTION]

In view of this situation, the present inventors made investigations with the aim of developing an industrially advantageous method of producing trehalose, and found that basidiomycetous yeasts of the genus *Filobasidium* selectively accumulate large amounts of trehalose in their cells. Trehalose accumulation by any yeast of the genus *Filobasidium* has not been known. The inventors made further investigations based on this finding, and completed the present invention.

The present invention relates to a method of producing trehalose wherein a yeast of the genus *Filobasidium* capable of producing trehalose is cultured in a medium to produce and accumulate trehalose in the culture, which trehalose is then recovered from the culture. The method of the present invention produces trehalose more efficiently than by the conventional method using an ascomycetous yeast of the genus *Saccharomyces* or an ascomycetous yeast of the genus *Hansenula*. The present inventors have unexpectedly found that the basidiomycetous yeasts of the genus *Filobasidium* are higher than the ascomycetous yeasts in both cell yield and intracellular trehalose content, and therefore that the amount of trehalose accumulated in the culture broth is improved greatly. According to the present invention when the carbon source concentration of the medium is increased the above-mentioned advantages in the cell yield and trehalose content become more remarkable, and the basidiomycetous yeasts of the genus *Filobasidium* can accumulate trehalose in amounts 3 to 5 times as much as the ascomycetous yeasts can accumulate. In the ascomycetous yeasts, addition of a carbon source during cultivation results in a rapid decline in intracellular trehalose content. The content, even if it gradually recovers over time, does not exceed the previous level. On the other hand, the above-mentioned basidiomycetous yeasts undergo almost no decline in trehalose content and cell growth occurs with high cell yield. As a result, a marked difference appears between the two types of yeast in trehalose yield per unit volume of culture broth. In addition, in the process of trehalose extraction and purification from cells of basidiomycetous yeasts of the genus *Filobasidium* after completion of cultivation, trehalose can easily be extracted, and the purification procedure is simple because no sugar other than trehalose is found.

Any yeast can be used for the present invention, as long as it belongs to the genus *Filobasidium* and is capable of producing trehalose, including *Filobasidium floriforme* and *Filobasidium capsuligenum*. Preferred strains include *Filobasidium floriforme* IFO 1603, *Filobasidium floriforme* IFO 1915, *Filobasidium floriforme* IFO 1916, *Filobasidium capsuligenum* IFO 1119 and *Filohasidium capsuligenum* IFO 1185. In the present invention, *Filobasidium floriforme* is preferred. All these examplary strains are known strains listed in the LIST OF CULTURES, 8th edition, 1988, published by the Institute for Fermentation, Osaka, and are readily available therefrom. Also, any mutant strain derived from the above strains or any strain newly isolated from soil, plant etc. can be used for the production method of the present invention, as long as it belongs to the genus *Filobasidium* and is capable of producing trehalose. The capability of producing trehalose can be checked by a per se known method, for example, the method described in working examples given later. Specific examples of the mutants include *Filobasidium floriforme* P87 (IFO 10614, FERM BP-4168) and *Filobasidium* floriforme T12 (IFO 10615, FERM BP-4167) derived from the parent strain, *Filobasidium floriforme* IFO 1916, for example, by UV-ray irradiation. Strain P87 is a mutant with lower productivity of extracellular polysaccharide, and, strain T12 is a trehalose non-assimilating mutant.

For conducting the process of the present invention, these yeast strains are cultured by conventional means. The means include batch culture, fed-batch culture or continuous culture under aerobic conditions such as shaking culture and submerged culture. Any medium can be used, as long as it is of an ordinary composition allowing the growth of the yeast used; various carbon sources and nitrogen sources can be selected. In addition to these additives, it is recommended that inorganic salts, amino acids, vitamins and, if desired other essential growth factors and growth promoters can be added. Examples of carbon sources include glucose, maltose, sucrose, fructose, starch, raw sugar, molasses and other sugars, glycerol, sorbitol and other sugar alcohols, and, conventional various organic acids, which may be used singly or in combination. These carbon sources may be added to the medium at initiation of cultivation to obtain the desired concentration, or may be added intermittently during cultivation. The sugar content is not critical, but is usually ranges from about 1.0 to about 30 w/v %. As shown in Examples given below, better results are obtained with higher carbon source concentrations by batch culture or fed-batch culture.

Examples of nitrogen sources include peptone, soybean flour, corn steep liquor, yeast extracts, meat extracts, urea and other organic nitrogen sources, and ammonium salts of sulfuric acid, nitric acid, hydrochloric acid and carbonic acid, aqueous ammonia, ammonia gas and other inorganic nitrogen sources, which may be used singly or in combination.

Examples of inorganic salts include sulfates, hydrochlorides, carbonates, nitrates, phosphates and acetates of calcium, potassium, sodium, magnesium, manganese, iron, copper and zinc, which may be used singly or in appropriate combination, if desired.

Because the yeasts of the genus *Filobasidium* used for the present invention require thiamine for growth, thiamine is an essential medium component. For this reason, the medium is supplemented with thiamine hydrochloride and other kinds of thiamine salt, thiamine-containing microbial cells or extracts thereof, meat extracts and other naturally occurring organic substances. The thiamine concentration of the medium is normally 0.1 to 5 mg/l, preferably 0.2 to 2 mg/l. In addition to these substances, various vitamins and amino acids may be added, when desired, for growth promotion. It is also effective to add antifoaming agent, such as silicon oil, to the medium at initiation of, or during, cultivation to prevent foaming.

Cultured broth pH normally falls in the range from 2 to 9, preferably from 3 to 7. To keep the pH in this range, phosphate buffer or calcium carbonate may be added to the medium at initiation of cultivation. The pH may be adjusted by adding alkali hydroxide, aqueous ammonia, ammonia gas or the like when the pH declines to the specified lower limit during cultivation, or by adding a mineral acid such as hydrochloric acid or sulfuric acid or an organic acid such as acetic acid or citric acid when the pH rises to the specified upper limit.

Culture temperature is selected within the range from 18 to 35°C, more preferably from 24 to 32°C, as appropriate to the growth of the yeast strain used and the intracellular accumulation of trehalose. Although it is common practice to conduct the cultivation at constant temperature within the above range, it may be sometimes effective to shift toward higher or lower temperature within the above range during cultivation.

Cultivation is continued until the amount of trehalose accumulated per unit volume of culture broth has reached maximum, normally in 24 to 120 hours.

The trehalose produced in the yeast cells can easily be recovered by combining known ordinary methods of purification, such as cell separation, extraction, chromatography and crystallization. Specifically, cell separation from the culture broth can be achieved by centrifugation using a yeast separator or by filtration using a filter press, ultrafilter or ceramic filter. Then, trehalose extraction from harvested cells can be achieved by solvent extraction. The solvent used to extract trehalose from the cells obtained is preferably (i) water, (ii) a dilute solution of perchloric acid or trichloroacetic acid, or (iii) a hydrophilic solvent such as ethanol, methanol or acetone or an aqueous solution thereof. Extraction is conducted by suspending the cells in the above solvent, agitating the mixture and recovering the solvent layer through filtration. Extraction may be made under heating, for example to 50 to 100°C. A particularly preferable method is the method in which cells are suspended in a 50 to 90 v/v% ethanolic aqueous solution and extracted while heating, or the method in which cells are suspended in a dilute solution of perchloric acid or trichloroacetic acid and extracted. Next, the thus-obtained extract, after concentration, if desired, is subjected to column chromatography using activated charcoal or cation and/or anion exchange resin to collect the trehalose fraction, which is then concentrated to a trehalose concentration of 40 to 60 w/v%. To the concentrate ethanol is added to make the final concentration of ethanol to be 70 to 90 v/v%, and the trehalose is crystallized. Crystallization can also be achieved from water depending on solubility differences. If the resulting coarse crystals are qualitatively insufficient, they may be redissolved in water and then recrystallised in the same manner as above to yield purified crystals. White crystals of trehalose dihydrate can be thus obtained.

The method of the present invention produces trehalose, an industrially useful substance, on an industrial scale at low cost. Specifically, cells selectively containing large amounts of trehalose can be obtained at very high yield by culturing a basidiomycetous yeast of the genus *Filobasidium* in a medium. Moreover, since trehalose can easily be extracted from the cells, and since there are almost no sugars or sugar alcohols other than trehalose in the cells, the present method permits simple purification and reproductive trehalose production at high yield. Furthermore, by using mutants derived from the said yeast, trehalose can be produced even more efficiently.

The present invention is hereinafter described in more detail by means of the following examples, which are not to be construed as limitative to the scope of the invention.

Example 1

To a 200 ml baffled flask containing 20 ml of the seed medium shown in Table 1, a loopful of *Filobasidium floriforme* IFO 1603 grown on agar slant medium (0.3 w/v% malt extract, 0.3 w/v% yeast extract, 0.5 w/v% peptane, 1.0 w/v% glucose and 1.5 w/v% agar) was inoculated, followed by 24 hours of cultivation at 24°C on a rotary shaker (200 rpm). A 1 ml aliquot of this seed culture broth was transferred to a 200 ml baffled flask containing 20 ml of main medium A shown in Table 1, followed by cultivation at 28°C on a rotary shaker (200 rpm). After 24 hours, 2 ml of a 18 w/v% glucose aqueous solution was added, and cultivation was continued further 24 hours at 28°C. In the course of cultivation, samples were taken periodically and assayed for dry cell weight in the culture broth and amount of trehalose accumulated per ml of the culture broth was calculated by the methods described below, respectively. The results are given in Table 2-1. The amount of trehalose accumulated reached a maximum (3.5 mg/ml) at 36 hours of cultivation. Since the dry cell weight was 17.5 mg/ml at that time, the trehalose content of dry cells was calculated as 20.0 w/w%. Separately, baker's yeast [isolated from the dry baker's yeast "Fermipan" (trade name), produced by Gist-brocades, Netherlands] and *Kluyveromyces thermotolerans* IFO 662, an ascomycetous yeast, were cultured in the same manner as above. As shown in Tables 2-2 and 2-3, in any case, the amount of trehalose accumulated was low, being half that accumulated by *Filobasidium floriforme*. Assays for dry cell weight and trehalose content were conducted as follows. Specifically, cells were collected by centrifugation from the culture broth (5 ml), and were washed twice by centrifugation with distilled water (5 ml) and then dried at 80°C to constant weight. Separately, to a centrifugation tube containing washed cells obtained in the same manner as above, 70 v/v% ethanol (4 ml) was added, and after vigorous stirring, the tube was stopped tightly and kept standing in a boiling water bath for 15 minutes. After cooling to room temperature, the cell residue was removed by centrifugation, and the resulting supernatant was assayed for trehalose content by high performance liquid chromatography [column, Shodex Sugar SZ 5532; eluent, acetonitrile : water = 80 : 20 (v/v); flow rate, 1 ml/min; temperature, 50°C; detector, differential refractometer].

EP 0 556 838 A1

Table 1

| Medium component | Seed medium | Main medium A | Main medium B | Main medium C |
|---|---|---|---|---|
| Glucose[1] | 1.0% | 1.8% | 5.4% | 10.0% |
| Malt extract | 0.3% | | | |
| Yeast extract | 0.3% | | | |
| Peptone | 0.5% | | | |
| CSL[2] | | | | 1.0% |
| YNB[3] | | 0.67% | 0.67% | |
| $KH_2PO_4$ | | 4.1% | 4.1% | 0.2% |
| $K_2HPO_4$ | | 5.2% | 5.2% | |
| $MgSO_4 \cdot 7H_2O$ | | | | 0.05% |
| $MnSO_4 \cdot 4\text{-}6H_2O$ | | | | 0.4mg/ml |
| Thiamine hydrochloride | | | | 0.4mg/ml |
| Antifoaming agent[4] | | | | 0.01% |
| Initial pH | Not adjusted | 6.0 | 6.0 | 5.0 |
| Percent figures are w/v%. | | | | |

1) Separately sterilized
2) Corn steep liquor
3) Yeast nitrogen base (produced by DIFCO)
4) Actocol distributed by Takeda Chemical Industries

Table 2-1

| (*Filobasidium floriforme* IFO 1603) | | | | |
|---|---|---|---|---|
| Culturing time (hr) | Dry cell weight (mg/ml) | Trehalose content (w/w%) | Amount of trehalose accumulated (mg/ml) | Residual sugar content[1] (mg/ml) |
| 24[2] | 9.0 | 14.5 | 1.30 | 0.01 |
| 30 | 14.0 | 20.0 | 2.80 | 0.20 |
| 36 | 17.5 | 20.0 | 3.50 | 0.00 |
| 48 | 16.5 | 20.0 | 3.30 | 0.00 |

1) Glucose
2) Immediately before secondary addition of glucose

Table 2-2

| (Baker's yeast) | | | | |
|---|---|---|---|---|
| Culturing time (hr) | Dry cell weight (mg/ml) | Trehalose content (w/w%) | Amount of trehalose accumulated (mg/ml) | Residual sugar content[1] (mg/ml) |
| 24[2] | 6.0 | 8.0 | 0.48 | 0.03 |
| 30 | 11.0 | 1.5 | 0.17 | 0.10 |
| 36 | 12.5 | 6.5 | 0.83 | 0.01 |
| 48 | 14.5 | 10.0 | 1.45 | 0.00 |

1) Glucose
2) Immediately before secondary addition of glucose

5

Table 2-3

| (*Kluyveromyces thermotolerans* IFO 662) | | | | |
|---|---|---|---|---|
| Culturing time (hr) | Dry cell weight (mg/ml) | Trehalose content (w/w%) | Amount of trehalose accumulated (mg/ml) | Residual sugar content[1] (mg/ml) |
| 24[2] | 7.5 | 15.0 | 1.13 | 0.01 |
| 30 | 8.5 | 6.5 | 0.55 | 0.15 |
| 36 | 9.5 | 10.0 | 0.95 | 0.01 |
| 48 | 9.5 | 12.0 | 1.14 | 0.00 |

1) Glucose

2) Immediately before secondary addition of glucose

## Example 2

Seed cultures of *Filobasidium floriforme* IFO 1603, IFO 1915 and IFO 1916 were prepared in accordance with the method described in Example 1, respectively.

A 1 ml aliquot of each culture broth was transferred to a 200 ml baffled flask containing 20 ml of main medium B shown in Table 1, followed by 48 hours of cultivation at 28°C on a rotary shaker (200 rpm). After completion of cultivation, the dry cell weight and trehalose content were determined by the method described in Example 1. The results, together with those from baker's yeast, sake yeast IFO 2376 and *Kluyveromyces thermotolerans* IFO 662, are given in Table 3. As seen in Table 3, the amount of trehalose accumulated by the yeasts of the genus *Filobasidium* were 4 to 10 times as high as those accumulated by the other ascomycetous yeasts.

Table 3

| Strain | Dry cell weight (mg/ml) | Trehalose content (w/w%) | Amount of trehalose accumulated (mg/ml) |
|---|---|---|---|
| *Filobasidium floriforme* IFO 1603 | 26.5 | 20.2 | 5.36 |
| *Filobasidium floriforme* IFO 1915 | 20.1 | 18.6 | 3.75 |
| *Filobasidium floriforme* IFO 1916 | 20.0 | 21.0 | 4.19 |
| Baker's yeast[1] | 9.8 | 10.2 | 0.99 |
| Sake yeast IFO 2376 | 9.5 | 5.7 | 0.54 |
| *Kluyveromyces thermotolerans* IFO 662 | 9.5 | 7.6 | 0.77 |

1) Isolated from the dry baker's yeast "Fermipan" (trade name), produced by Gist-brocades, Netherlands

## Example 3

Culture broth (10 ml) of *Filobasidium floriforme* IFO 1916 as obtained in Example 2 was poured into a centrifugal tube. Cells were collected and washed twice with distilled water (5 ml) by centrifugation. To the washed cells, 10 ml of 70 v/v% ethanol was added. After vigorous stirring, the tube was stopped tightly and kept standing in a boiling water bath for 15 minutes. Next, the supernatant was separated from the cell residue by centrifugation. Cell residue was washed with 6 ml of 70 v/v% ethanol by centrifugation. These supernatant were combined and made up to 20 ml. This solution was found to contain 2.10 mg/ml trehalose. Separately, the same experiment as above was conducted using distilled water in place of 70 v/v% ethanol; the extract was found to contain 2.04 mg/ml trehalose, equivalent to 97% of the value obtained with 70 v/v% ethanol.

Example 4

A 125 ml portion of a seed culture broth of *Filobasidium floriforme* IFO 1916, prepared in accordance with the method of Example 1, was transferred to a 5 liter jar fermentor containing 2.5 liter of main medium C shown in Table 1, followed by cultivation at 28°C by submerged culture with stirring at 800 rpm and at an aeration rate of 1.25 liter/min. During cultivation, an 15 w/v% sodium hydroxide solution was added to maintain the pH at 5.0. After 27 hours cultivation, glucose had been thoroughly consumed, and the dry cell weight and amount of trehalose accumulated were determined, by the methods described in Example 1, to be 49.5 mg/ml and 10.0 mg/ml, respectively. The intracellular trehalose content was thus 20.2 w/w%.

From this culture broth, trehalose was purified as follows: First, 1 liter of the cultured broth was centrifuged (5000 X g, 15 minutes) to collect cells, which were washed with 500 ml of distilled water. To the washed cells was added 1 liter of 70 v/v% ethanol, and the flask was kept standing in a boiling water bath for 15 minutes with stirring. This suspension was centrifuged to yield a clear supernatant.

After the ethanol was distilled off under reduced pressure, the extract (320 ml) was applied to a column (3 X 30 cm) of activated charcoal (LH$_2$C charcoal, produced by Takeda Chemical Industries). The column was washed with about 300 ml of distilled water, and trehalose was eluted with 10 v/v% ethanol. The trehalose-containing fraction (550 ml) was collected and concentrated to 12 ml by distillation under reduced pressure. To this concentrate, ethanol was gradually added to a final concentration of 80 v/v% with stirring, after which the mixture was left at 4°C overnight. The crystals were collected by filtration, washed with a small amount of ethanol and dried at 60°C for 5 hours. Finally, 7.9 g of white crystal was obtained.

The optical rotation of the crystal was determined to be $[\alpha]_D^{29.1} = + 176.3$, and differential thermal analysis revealed a water content of 9.24% and a melting point of 213.2°C. These values agreed well with those of the control trehalose dihydrate, produced by Sigma (USA) [D( +) trehalose dihydrate, crystalline, Product Number T5251]. The infrared absorption spectrum of the crystal agreed very well with that of the trehalose of Sigma. These findings have identified this crystal as the dihydrate of trehalose (O-$\alpha$-D-glucopyranosyl-(1→1)-$\alpha$-D-glucopyranoside).

Example 5

A 125 ml portion of a seed culture broth of *Filobasidium floriforme* IFO 1916, prepared in accordance with the method of Example 1, was transferred to a 5 liter jar fermentor containing 2.5 liter of main medium C shown in Table 1, followed by cultivation at 28°C by submerged culture with stirring at 800 rpm and at an aeration rate of 1.25 liter/min. During cultivation, an 15 w/v% sodium hydroxide solution was added to maintain the pH at 5.0. After 16 hours from the initiation of cultivation, the temperature was shifted up to 32°C and the cultivation was continued. After 33 hours from the initiation of cultivation, the glucose was found to have been thoroughly consumed. The dry cell weight and the amount of trehalose accumulated were determined in accordance with the method described in Example 1 to be 42.3 mg/ml and 11.0 mg/ml, respectively. The intracellular trehalose content was thus 26.0 w/w%.

Example 6

Twenty ml of a seed medium shown in Table 1 contained in a 200 ml baffled flask was inoculated with one loopful of *Filobasidium floriforme* IFO 1916 grown on an agar slant culture medium (0.3 w/v% malt extract, 0.3 w/v% yeast extract, 0.5 w/v% peptone, 1.0 w/v% glucose and 1.5 w/v% agar), followed by incubation at 24°C for 24 hours on a rotary shaker (200 rpm). Cells were collected by centrifugation and washed once with a 0.1 M potassium phosphate buffer solution (pH 7.0), and then suspended in the same buffer solution to make the cell density to be about $4 \times 10^6$/ml. Ten ml of this suspension was put in a sterilized Petri dish, which was subjected to UV irradiation (for about 5.5 min.) by using a U.V. lamp (Toshiba, 15W) at a distance of about 30 cm so that the rate of living cells was reduced to about 1%. The irradiated suspension was diluted to $10^2$, $10^3$ and $10^4$ times. Each sample was spread on a YMG agar plate culture medium (0.3 w/v% maltose, 0.3 w/v% yeast, 0.5 w/v% peptone, 2.0 w/v% glucose and 2.0 w/v% agar), followed by incubation at 24°C for 3 days. While almost all the colonies were highly mucous and glistening like the parent strain, a very few colonies appeared which had different morphology from that of the parent strain, i.e. non-mucous and non-glistening. These strains were picked up and their properties were compared with those of parent strain, by the method described later. These strains were found to produce less extracellular polysaccharide and more intracellular trehalose. Of these mutants, a typical strain was selected and designated strain P87. Strain P87 was cultured by using a jar fermentor in accordance with the method described in Example 4. After completion of the cultivation, the amount of dry cells and the content of trehalose were determined in accordance with the method described in Example 1. The amount of extracellular polysaccharide accumulated in the supernatant of the culture broth was determined as follows. To the supernatant (5 ml) was added 4 volumes of ehtanol. The resulting white precipitates were collected centrifugally, and washed once with 80v/v% ethanol (5 ml), and dissolved in 5ml of distilled water. The total sugar in this aqueous solution was determined by means of the phenol-sulfuric acid method using glucose as the standard. Table 4 shows results of the determination.

Table 4

| Strain | Dry cell weight (mg/ml) | Extracellular polysaccharide (mg/ml) | Trehalose content (w/w%) | Amount of trehalose accumulated (mg/ml) |
|---|---|---|---|---|
| P87 | 48.5 | 0.84 | 26.0 | 12.6 |
| 1916 | 49.5 | 4.45 | 20.2 | 10.0 |

As is apparent from this Table, the amount of extracellular polysaccharide produced by strain P87 was less than 1/5 and that of trehalose accumulated was 126%, in comparison with the parent strain.

Example 7

From one liter of the cultured broth obtained in Example 6, trehalose was recovered in accordance with the method described in Example 4. Finally 10.0 g of white crystals was obtained. As compared with the case of employing the parent strain (Example 4), the yield increased by 27%. And, in the case of employing strain P87, the viscosity of the culture broth is lowered, though the dry cell weight is substantially the same as in the case of the parent strain, and the cell volume after centrifugation also decreased to about 1/2 relative to the case of the parent strain. This served not only to facilitate the recovery of cells, but also to decrease, to a great extent, the volume of ethanol required for extracting trehalose from the cells. As a result, the purification process was remarkably improved.

Example 8

*Filobasidium floriforme* IFO 1916 was mutated by substantially the same procedure as in Example 6. The irradiated suspension was diluted to $10^2$, $10^3$ and $10^4$ times, respectively. Each cell suspension was spread on YMG agar plate (maltose extract 0.3 w/v %, yeast extract 0.3 w/v%, peptone 0.5 w/v%, glucose 2.0 w/v% and agar 2.0 w/v%), and incubated for two days to allow colonies to grow. These colonies were transferred, by means of replica plating technique, to a YNBPT agar plate (YNB 0.67 w/v%, $KH_2PO_4$ 1.37 w/v%, $K_2HPO_4$ 1.73 w/v%, trehalose 0.5 w/v%, agar 1.5 w/v%; pH 6.0) and a YNBPG agar plate (YNB 0.67 w/v%, $KH_2 PO_4$ 1.37 w/v%, $K_2HPO_4$ 1.73 w/v%, glucose 0.5w/v%, agar 1.5 w/v%; pH 6.0), followed by incubation at 24°C for two days. In this way, several strains, which grew on a YNBPG agar plate, but did not on a YNBPT agar, were obtained. One loopful of each strain was inoculated on a YNBPT liquid culture medium and a YNBPG liquid culture medium, followed by incubation at 24°C for 48 hours with shaking. The growth on the respective media was examined. These strains grew on the YNBPG liquid culture medium to substantially the same extent as the parent strain but hardly grew on the YNBPT liquid culture medium. Of these strains, one typical strain was selected and designated strain T12. Growth of strain T12 is shown in Table 5. Strain T12 was a mutant which was substantially deficient in the ability of assimilating trehalose. And, the amount of trehalose accumulated was examined in accordance with the procedure described in Example 4. As shown in Table 6, it increased by 36% relative to the parent strain.

Table 5

| Strain | Growth (UOD$_{570}$)[1] | |
|---|---|---|
| | YNGB culture medium | YNBT culture medium |
| T12 | 6.74 | 0.62 |
| 1916 | 6.76 | 6.68 |

1) turbidity of diluted solution at 570 nm x dilution multiple

Table 6

| Strain | Drycell weight (mg/ml) | Trehalose content (w/w%) | Amount of trehalose accumulated (mg/ml) |
|---|---|---|---|
| T12 | 49.7 | 27.4 | 13.6 |
| 1916 | 49.5 | 20.2 | 10.0 |

**Claims**

1. A method of producing trehalose, which comprises cultivating in a medium a yeast of the genus *Filobasidium* capable of producing trehalose to allow trehalose to be accumulated in the culture and recovering trehalose from the culture.

2. The method claimed in Claim 1, wherein the yeast of the genus *Filobasidium* capable of producing trehalose is a yeast belonging to the species *Filobasidium floriforme* capable of producing trehalose.

3. The method claimed in Claim 1, wherein the yeast of the genus *Filohasidium* capable of producing trehalose is *Filobasidium floriforme* IFO 1603.

4. The method claimed in Claim 1, wherein the yeast of the genus *Filobasidium* capable of producing trehalose is *Filobasidium floriforme* IFO 1915.

5. The method claimed in Claim 1, wherein the yeast of the genus *Filobasidium* capable of producing trehalose is *Filobasidium floriforme* IFO 1916.

6. The method of producing trehalose claimed in Claim 1, wherein the yeast of the genus *Filobasidium* capable of producing trehalose is a mutant with low productivity of extracellular polysaccharide.

7. The method of producing trehalose claimed in Claim 6, wherein the mutant is *Filobasidium floriforme* P87.

8. The method of producing trehalose claimed in Claim 1, wherein the yeast of the genus *Filobasidium* capable of producing trehalose is a trehalose non-assimilating mutant.

9. The method of producing trehalose claimed in Claim 1, wherein the mutant is *Filobasidium floriforme* T12.

10. *Filobasidium floriforme* P87.

11. *Filobasidiuin floriforme* T12.

9

## DOCUMENTS CONSIDERED TO BE RELEVANT

EP 93102590.2

| Category | Citation of document with indication, where appropriate, of relevant passages | Relevant to claim | CLASSIFICATION OF THE APPLICATION (Int. Cl.5) |
|---|---|---|---|
| D,A | THE JOURNAL OF THE AMERICAN CHEMICAL SOCIETY, vol. 72, 1950, New York<br>L.C. STEWART et al. "The Preparation of Trehalose from Yeast",<br>pages 2059-2061<br>* Totality * | 1 | C 12 P 19/12<br>C 12 N 1/16 |
| D,A | DD - A - 266 584 (AKADEMIE DER WISSENSCHAFTEN DER DDR)<br>* Claims * | 1 | |
| A | EP - A - 0 451 896 (GIST-BROCADES)<br>* Abstract * | 1 | |
| A | CHEMICAL ABSTRACTS, vol. 112, no. 23, June 04, 1990 Columbus, Ohio, USA<br>E. GUEHO et al. "Evolutionary affinities of heterobasidomycetous yeasts estimated from 18S and 25S ribosomal RNA sequence divergence",<br>page 153, right column, abstract-no. 211 790b<br>& Syst. Appl. Microbiol. 1989, 12(3), 230-6 | 10,11 | TECHNICAL FIELDS SEARCHED (Int. Cl.5)<br><br>C 12 P<br>C 12 N |

The present search report has been drawn up for all claims

| Place of search | Date of completion of the search | Examiner |
|---|---|---|
| VIENNA | 28-05-1993 | WOLF |